# EUROPEAN PATENT SPECIFICATION

(11) **EP 3 386 526 B1**
(45) Date of publication and mention of the grant of the patent: **13.11.2024**
(21) Application number: 16871873.2
(22) Date of filing: 09.12.2016
(51) Int. Cl.: A61K 36/45, A61P 31/04, A61K 31/505, A61K 31/635, A61K 31/352, A61K 31/7036, A61K 31/65, A61K 31/7052, A61K 31/4178, A61K 31/665, A61K 31/496, A61K 31/43, A61K 45/06

(54) **USE OF CRANBERRY DERIVED PHENOLIC COMPOUNDS AS ANTIBIOTIC SYNERGIZING AGENT AGAINST PATHOGENIC BACTERIA**
VERWENDUNG VON AUS MOOSBEEREN GEWONNENEN PHENOLVERBINDUNGEN MIT ANTIBIOTIKA ALS SYNERGIE-MITTEL GEGEN PATHOGENE BAKTERIEN
UTILISATION DE COMPOSÉS PHÉNOLIQUES DÉRIVÉS DE CANNEBERGE EN TANT QU'AGENT DE SYNERGIE ANTIBIOTIQUE CONTRE DES BACTÉRIES PATHOGÈNES

(30) Priority: 11.12.2015 US 201562266334 P; 26.07.2016 US 201662366666 P
(43) Date of publication of application: 17.10.2018
(73) Proprietor: The Royal Institution for the Advancement of Learning / McGill University, Montreal, Quebec H3A 0G4 (CA)
(72) Inventor: TUFENKJI, Nathalie, Laval, Québec H7Y 2H8 (CA); MAISURIA, Vimal Bharatbhai, Montréal, Québec H2X 2C8 (CA)
(74) Representative: Germain Maureau
(86) International application number: PCT/CA2016/051447
(87) International publication number: WO 2017/096484

(56) References cited:
- WO-A1-2010/078660
- WO-A2-2007/099409
- US-A1- 2012 214 781
- J. UBEROS ET AL: "Phenolic acid content and antiadherence activity in the urine of patients treated with cranberry syrup (Vaccinium macrocarpon) vs. trimethoprim for recurrent urinary tract infection", JOURNAL OF FUNCTIONAL FOODS, vol. 18, 1 October 2015 (2015-10-01), NL, pages 608 - 616, XP055487422, ISSN: 1756-4646, DOI: 10.1016/j.jff.2015.08.009
- ULREY, R. K. ET AL.: "Cranberry proanthocyanidins have anti-biofilm properties against Pseudomonas aeruginosa", BMC COMP. ALTERN. MED., vol. 14, 2014, pages 499, XP021208009, ISSN: 1472-6882
- DIARRA, M. S. ET AL.: "In vitro and in vivo antibacterial activities of cranberry press cake extracts alone or in combination with beta-lactams against Staphylococcus aureus", BMC COMP. ALTERN. MED., vol. 13, 2013, pages 90, XP021148214, ISSN: 1472-6882
- DOSLER , S. ET AL.: "Inhibition and destruction of Pseudomonas aeruginosa biofilms by antibiotics and antimicrobial peptides", PEPTIDES, vol. 62, 5 October 2014 (2014-10-05), pages 32 - 37, XP029099176, ISSN: 0196-9781
- HOWELL, A. B. ET AL.: "A-type cranberry proanthocyanidins and uropathogenic bacterial anti-adhesion activity", PHYTOCHEM., vol. 66, no. 18, September 2005 (2005-09-01), pages 2281 - 91, XP002507858, ISSN: 0031-9422

## Description

### CROSS-REFERENCE TO RELATED APPLICATIONS

The present application claims benefit of U.S. Provisional Application No. 62/266,334 filed December 11, 2015, and of U.S. Provisional Application No. 62/366,666 filed July 26, 2016.

### TECHNICAL FIELD

The present description relates to a synergistically active composition comprising a cranberry extract and at least one antibiotic for treating a bacterial infection.

### BACKGROUND ART

As antibiotic resistance in microbial pathogens embodies a global threat to public health, it demands the development of novel strategies for managing microbial infections. The long-term effectiveness of most antibiotic treatments is restricted by both pathogen drug resistance and non-target effects on the host's commensal microbial community. Over the last decade, research on antimicrobials has shifted towards an alternative approach to combat pathogens using anti-infective drugs that selectively interrupt virulence pathways to help prevent or cure bacterial infections. Anti-infective drugs that do not perturb survival or viability of bacterial pathogens should be less likely to promote resistance than conventional antibiotics. Until now, the development of anti-infective synthetic drugs has been limited to the laboratory and preclinical studies. Natural bioactive compounds derived from plant species show promising therapeutic properties to combat the emerging resistance in microbial pathogens, which can be exploited as next generation anti-infective drugs.

The identification of selective anti-virulence therapies that abolish the production of virulence determinants without affecting the viability of pathogenic bacteria would be extremely useful in combating bacterial infections caused by broad-spectrum antibiotic-resistant pathogens.

There is thus a need to be provided with new antibacterial composition.

### SUMMARY

In accordance with the present disclosure, there is now provided a synergistically active composition according to claim 1.

In an embodiment, the cranberry extract comprises proanthocyanidins, flavanols, anthocyanidins, procyanidins, terpenes, hydroxybenzoic acids, hydroxycinnamic acids, flavonoids, tannins, phenolic acids, other bioactive molecules or combinations thereof.

In another embodiment, the cranberry extract is from at least one of *Vaccinium macrocarpon, Vaccinium oxycoccos, Vaccinium microcarpum,* and *Vaccinium erythrocarpum.*

In a further embodiment, the cranberry extract is from *Vaccinium macrocarpon.*

In another embodiment, at least one antibiotic is an aminoglycoside, a polyketide, a macrolide, a benzenoid, an azolidine, an organic phosphonic acid, or their derivatives and combinations thereof.

In an embodiment, at least one antibiotic is kanamycin tetracycline, azithromycin, trimethoprim, sulfamethoxazole, nitrofurantoin, fosfomycin, or their combinations thereof

In another embodiment, at least one antibiotic is trimethoprim and sulfamethoxazole.

In a further embodiment, the composition is a quorum sensing (QS) inhibitor.

In another embodiment, the composition permeabilizes cell membranes to the at least one antibiotic.

In a further embodiment, the composition inhibits efflux pumps.

In an embodiment, the composition enhances membrane transport of tetracycline.

In another embodiment, the composition described is an antagonist of LasR or RhIR.

It is also described the use of the composition described herein for treating a bacterial infection.

It is also described the use of the composition described herein in the manufacture of a medicament for treating a bacterial infection.

It is further described the use of the composition encompassed herein for decreasing multidrug resistance.

It is further described the use of the composition encompassed herein for decreasing biofilm formation.

It is also described a method of treating a bacterial infection, comprising administering the composition described herein.

In an embodiment, the subject is an animal or a human.

### BRIEF DESCRIPTION OF THE DRAWINGS

Reference will now be made to the accompanying drawings.

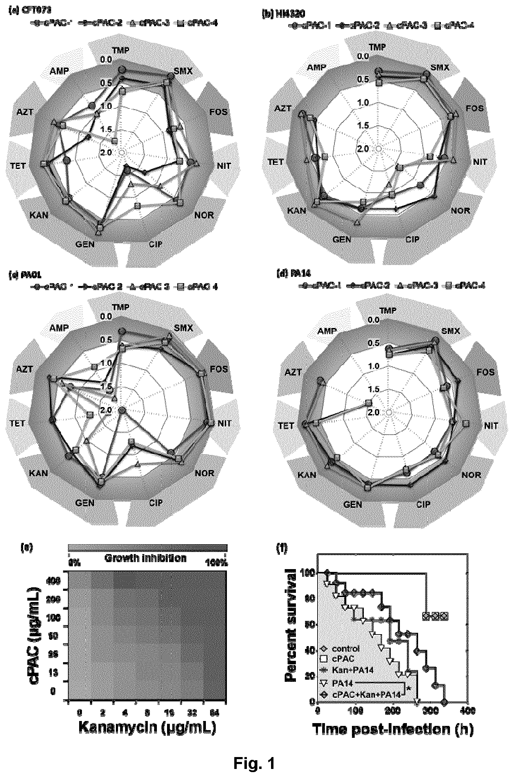

Fig. 1 illustrates the synergistic interaction of an extract rich in cranberry proanthocyanidin (cPAC) with an antibiotic for growth inhibition. MICs were determined for combination of cPAC fractions in combination with each antibiotic indicated with their classes. (A-D) Fractional inhibitory concentration (FIC) index for each combination against (A) E. *coli* CFT073, (B) *P. mirabilis* HI4320, (C) *P. aeruginosa* PAO1 and (D) P. *aeruginosa* PA14. FIC index of ≤0.5 indicated with gray color at border. (E) Representative heat plot showing synergistic growth inhibition of P. *aeruginosa* PA14 by kanamycin in combination with cPAC fraction. (F) *In vivo* synergy between kanamycin and cPAC fraction was tested in a D. *melanogasterfly* feeding model. Flies were infected by feeding with *P. aeruginosa* PA14 cells and maintained on agar containing kanamycin (16 µg mL⁻¹) alone and in combination with cPAC (200 µg mL⁻¹). Mortality was scored daily for 14 days. Results represent measurements from experiments performed with triplicates, twice (**P*< 0.05). TMP: trimethoprim; SMX: sulfamethoxazole; FOS: fosfomycin; NIT: nitrofurantoin; NOR: norfloxacin; CIP: ciprofloxacin; GEN: gentamicin; KAN: kanamycin; TET: tetracycline; AZT: azithromycin; AMP: ampicillin.

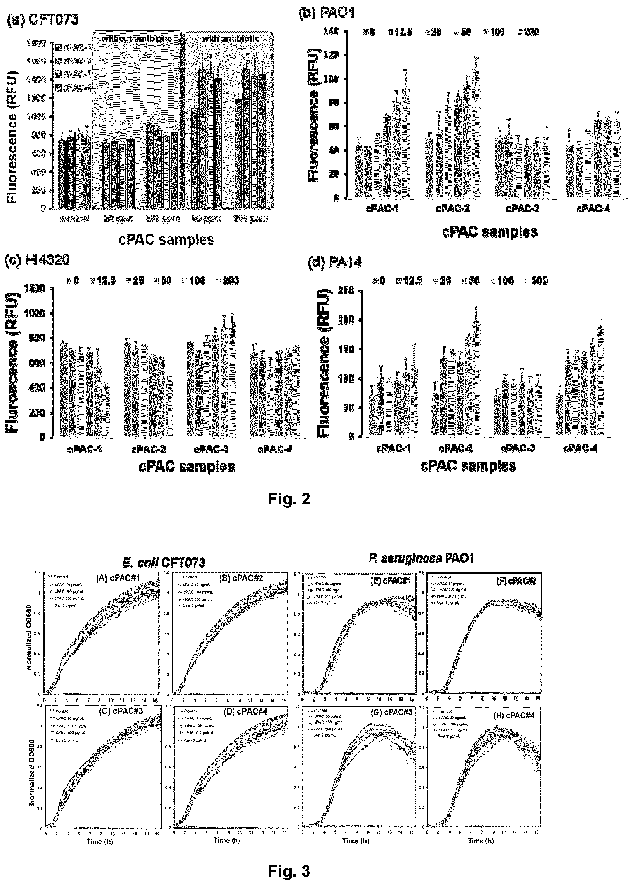

Fig. 2 illustrates the mechanisms of antibiotic-cPAC synergy. The cPAC mediated uptake of tetracycline in (A) *E*. *coli* CFT073, (B) *P. mirabilis* HI4320, (C) *P. aeruginosa* PAO1 and (D) *P*. *aeruginosa* PA14 cells. Concentration of tetracycline was 4 µg mL⁻¹ for CFT073 and HI4320I; 8 µg mL⁻¹ for PAO1 and PA14, and concentration of each cPACs sample was as indicated.
Fig. 3 illustrates growth curves for (A-D) *E*. *coli* CFT073 and (E-H) P. *aeruginosa* PAO1 with different cPAC samples or gentamycin. Bacteria grown in the presence of (A, E) cPAC#1, (B, F) cPAC#2, (C, G) cPAC#3, (D, H) cPAC#4 or gentamicin. The graph shows the normalized OD600 = OD600 - initial OD600 versus time for bacteria grown in MHB-II broth (control) or with cPAC alone (concentration as indicated) or with gentamicin (MIC 2 µg/mL) alone. Data shown in growth curves are averages of n=3 with shaded S.D.

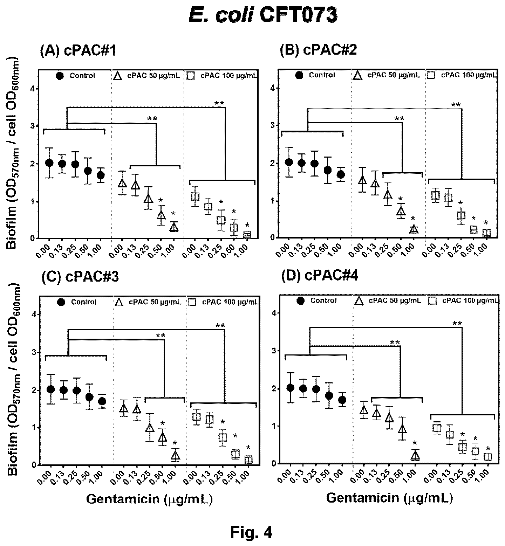

Fig. 4 illustrates the effect of each cPAC fraction with and without gentamicin on biofilm formation of *E*. *coli* CFT073. The graph presents normalized biofilm levels (OD₅₇₀nm/cell OD₆₀₀nm) versus different sub-inhibitory concentrations of gentamicin for *E*. *coli* CFT073 grown in MHB-II medium (control) or in MHB-II medium amended with sub-inhibitory concentrations of cPAC#1, cPAC#2, cPAC#3, or cPAC#4, with and without gentamicin. Error bars show the standard deviations from values obtained from three replications. Statistically significant differences are indicated for each sample treated with each cPAC fraction and gentamicin compared to the control (sample treated with the corresponding concentration of gentamicin only) (**, P <0.01; Two-way ANOVA) and also for samples treated with each cPAC fraction plus gentamicin compared to sample treated with the same concentration of each cPAC fraction without gentamicin (*, P<0.05; Two-way ANOVA).

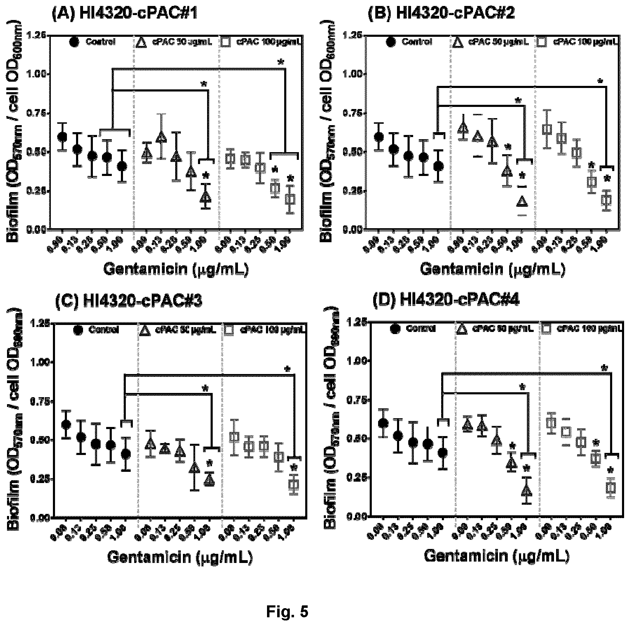

Fig. 5 illustrates the effect of cPAC alone and in combination with gentamicin on biofilm formation of *P. mirabilis* HI4320. The graph presents normalized biofilm levels (OD₅₇₀ₙₘ/cell OD₆₀₀ₙₘ) versus different sub-inhibitory concentrations of gentamicin. Statistically significant differences are indicated for each sample treated with each cPAC fraction and gentamicin compared to the control (sample treated with the corresponding concentration of gentamicin only) (*, P <0.05; Two-way ANOVA) and also for samples treated with each cPAC fraction plus gentamicin compared to sample treated with the same concentration of each cPAC fraction without gentamicin (*, P<0.05; Two-way ANOVA).

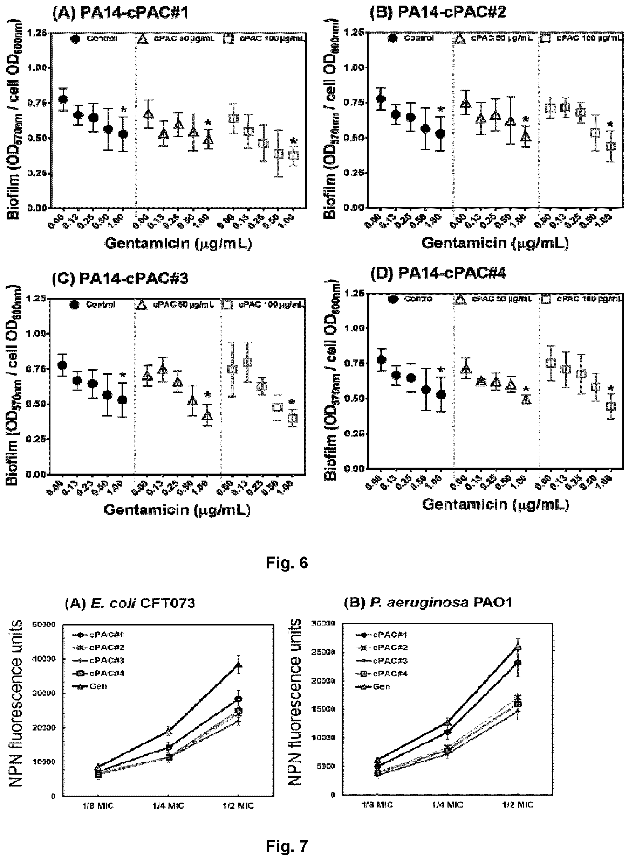

Fig. 6 illustrates the effect of cPAC alone and in combination with gentamicin on biofilm formation of *P. aeruginosa* PA14. The graph presents normalized biofilm levels (OD₅₇₀ₙₘ/cell OD₆₀₀ₙₘ) versus different sub-inhibitory concentrations of gentamicin. Statistically significant differences are indicated for samples treated with each cPAC fraction plus gentamicin compared to sample treated with the same concentration of each cPAC fraction without gentamicin (*, P<0.05; Two-way ANOVA).
Fig. 7 illustrates cPAC-mediated NPN uptake in (A) E. *coli* CFT073 and (B) *P. aeruginosa* PAO1. Bacterial cells were pretreated with cPAC#1, cPAC#2, cPAC#3, cPAC#4 or gentamicin (Gen) at sub-MICs. Enhanced uptake of NPN was measured by an increase in fluorescence (ex/em: 350 nm/420 nm) caused by partitioning of NPN into the hydrophobic interior of the outer membrane of pretreated bacterial cells. NPN is a hydrophobic fluorescent probe that fluoresces weakly in aqueous environment and strongly when it enters a hydrophobic environment such as the interior of a bacterial membrane. The background fluorescence of the medium was subtracted from all measurements, and the assay was repeated.

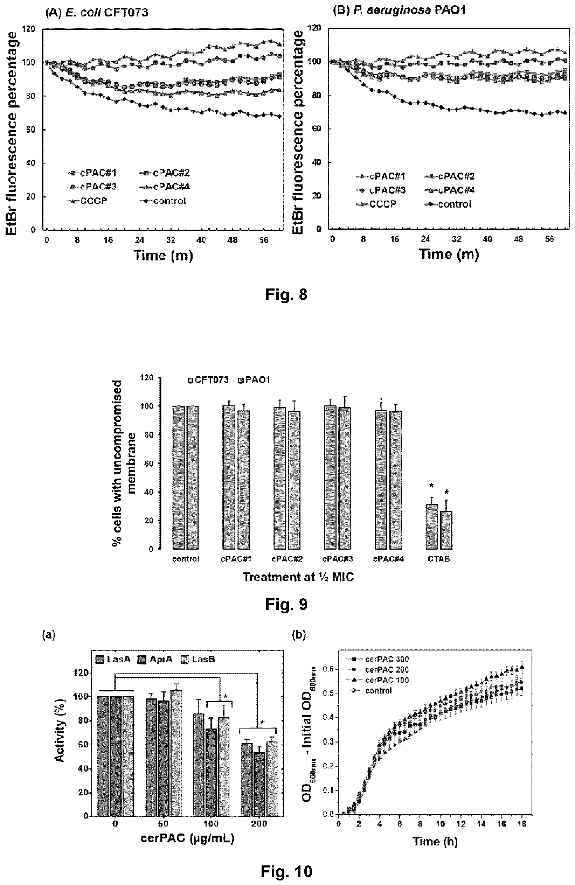

Fig. 8 illustrates the inhibition of multidrug efflux pump by cPACs in (A) *E. coli* CFT073 and (B) P. *aeruginosa* PAO1. Bacterial cells were pretreated without (control) and with 200 µg/mL cPAC#1, 200 µg/mL cPAC#2, 200 µg/mL cPAC#3, 200 µg/mL cPAC#4 or 100 µM CCCP (carbonyl cyanide m-chlorophenylhydrazone). EtBr efflux pump activity of the pretreated bacterial cells was monitored at room temperature for fluorescence intensity (ex/em: 530 nm/600 nm). Active efflux pump reduces accumulation of intracellular EtBr whereas inhibition of the efflux pump enhances accumulation of intracellular EtBr over time. The background fluorescence of the medium was subtracted from all measurements, and the assay was repeated independently in triplicate.
Fig. 9 illustrates the effect of each cPAC fraction on cell membrane integrity. Bacterial cells of E. *coli* CFT073 and *P. aeruginosa* PAO1 were pretreated separately with cPAC#1, cPAC#2, cPAC#3, cPAC#4 or cetyltrimethylammonium bromide (CTAB) at 1/2 MICs. The ratio of green to red fluorescence was normalized to that of the untreated control and expressed as a percentage of the control. The assay was repeated independently three times (*, P<0.05; student's t-test).
Fig. 10 illustrates in (A) the inhibition of virulence determinants and (B) growth curves of *P. aeruginosa* PA14 in absence or presence of different cranberry extract rich in proanthocyanidin (cerPAC) concentrations. LasA: staphylolytic protease, LasB: elastase and AprA: alkaline protease. Results are expressed as means and standard deviations (SD) of triplicate enzyme assays (*P< 0.001). Bacterial growth (OD₆₀₀) was monitored at 37 °C for 18 h in TSB medium. Error bars with average data points of growth kinetics represent the standard deviation of values obtained from four replicates. Abbreviations: cerPAC x, Cranberry extract rich in proanthocyanidins at x µg mL⁻¹ (e.g., cerPAC 300 indicates cerPAC at 300 µg mL⁻¹).

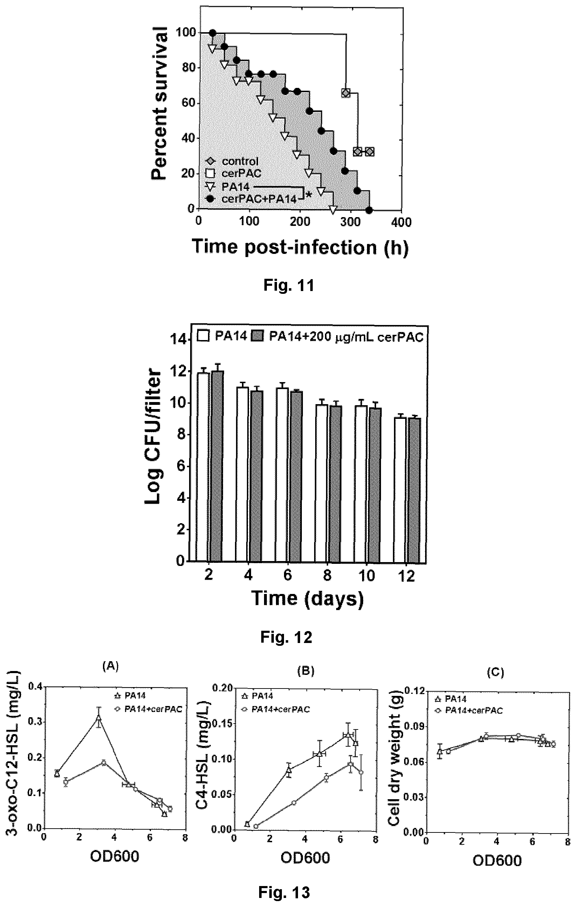

Fig. 11 illustrates the virulence of *P. aeruginosa* PA14 towards D. *melanogaster* in absence or presence of cerPAC (200 µg mL⁻¹). Mortality was scored daily for 14 days. Results represent measurements from experiments performed with triplicates, twice (*P < 0.05).
Fig. 12 illustrates the survival of *P. aeruginosa* PA14 on sucrose filters during the fly feeding assay. Total viable bacterial counts per filter were determined every second day for filters containing bacterial cells on sucrose agar. Average data represent data from triplicate assays and error bars represent S.D.
Fig. 13 illustrates that cerPAC (200 µg mL⁻¹) impairs the production of AHL-type QS molecules in *P. aeruginosa* PA14. Concentrations of (A) 3-oxo-dodecanoyl-homoserine lactone (3-oxo-C₁₂-HSL), and (B) butanoyl-homoserine lactone (C₄-HSL) are shown as a function of cell growth (OD₆₀₀). (C) Total cell dry weight of 3 mL culture is shown as a function of cell growth (OD₆₀₀). Data points represent the average of triplicate experiments and the error bars show the standard deviation.

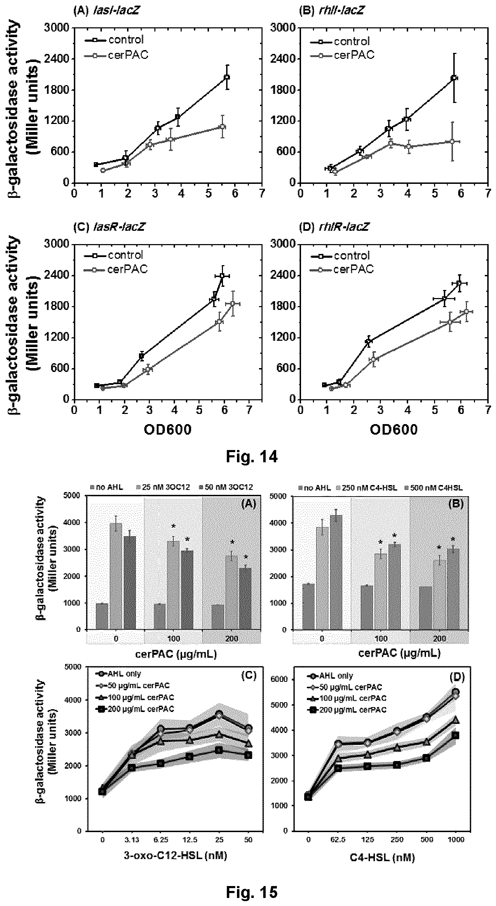

Fig. 14 illustrates the effect of cerPAC on the expression of quorum sensing genes. *P. aeruginosa* PA14 carrying reporter fusion plasmids (A) *lasI'-lacZ* (B) *rhlI'-lacZ,* (C) *lasR'-lacZ* and (D) *rhlR'-lacZ* were grown in TSB medium without or with 200 µg mL⁻¹ cerPAC, and expression was quantified by measuring β-galactosidase activity. Data points represent the average of triplicate experiments. The error bars show the standard deviation.
Fig. 15 illustrates that cerPAC represses AHL induction of LasR- and RhlR-controlled regulation in *P. aeruginosa* PA14. (A) LasR activation of *lasl-lacZ* activity in Δ*lasI-* mutant of PA14, (B) RhIR activation of *rhlI-lacZ* activity in Δ*rhll-*mutant of PA14. Titration for activation of (C) *lasI-lacZ* in Δ*lasI-* mutant of PA14 and (D) *rhlI-lacZ* in Δ*rhlI-* mutant of PA14 in absence and presence of cerPAC. Error bars (A, B) and shaded errors (C, D) represent SD of triplicate assays. Statistically significant differences are indicated for each sample treated with cerPAC and each autoinducer compared to the sample treated with the corresponding concentration of each autoinducer (*, *P* <0.05).

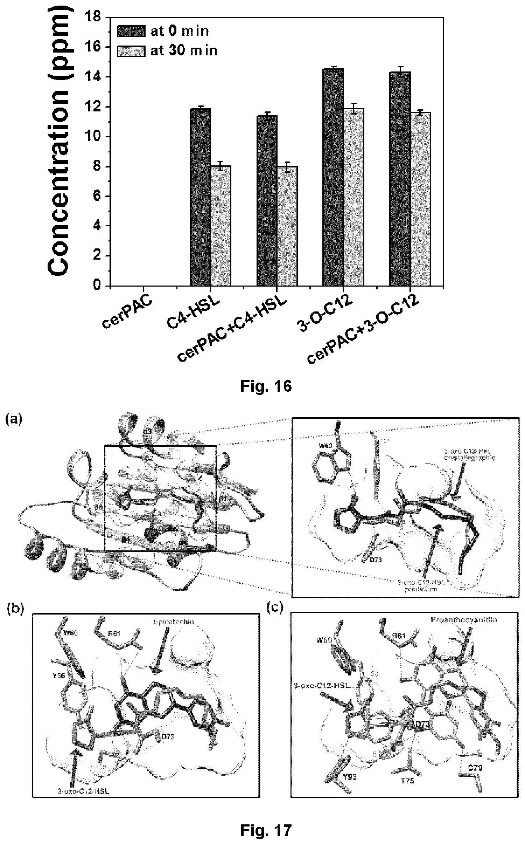

Fig. 16 illustrates the interaction of cerPAC with AHL molecules in TSB medium. 15 ppm C₄-HSL and 15 ppm 3-oxo-C₁₂-HSL (3-O-C12) were added individually in sterile TSB with or without 200 µg mL⁻¹ cerPAC, and concentrations were quantified by LC/MS. C₄-HSL and 3-oxo-C₁₂-HSL indicates only AHL controls. Data points are the average with standard deviation of triplicate assays.
Fig. 17 illustrates the molecular docking analysis of the LasR protein with AHL molecule and two main components of the cerPAC. (A) Left panel represents full view of the ribbon structure of LasR protein with the ligand binding cavity between four β-sheets (β1, β2, β4 and β5) and two α-helixes (α3 and α4). Upper right panel represents the inset view of docked complex with known binding position (reported crystallographic structure) of ligand 3-oxo-C12-HSL and the predicted binding position of 3-oxo-C12-HSL during *in silico* docking. The docking complexes of LasR with (B) the monomer of epicatechin and (C) the dimeric form of the epicatechin (proanthocyanidin) are shown in the presence of 3-oxo-C12-HSL for the comparison of binding positions. All possible hydrogen bonds are shown using black lines and binding residues shown.

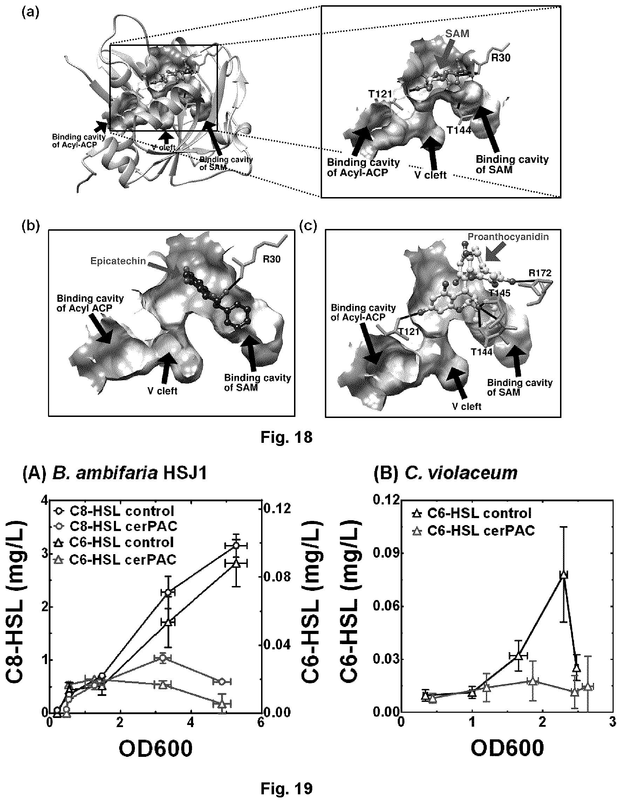

Fig. 18 illustrates the molecular docking analysis of the Lasl protein with substrate S-adenosyl L methionine (SAM) and two main components of the cerPAC. (A) Left panel represents full view of the ribbon structure of Lasl protein with its substrates binding cavities and right panel represents the inset view of docked complex with substrate SAM. The docking complexes of Lasl with (B) the monomer of epicatechin and (C) the dimeric form of the epicatechin (proanthocyanidin) are shown with predicted binding residues (shown in bright green color). The surface structures are shown for hydrophobic and hydrophilic attributes, respectively, and possible hydrogen bonds are depicted using black lines.
Fig. 19 illustrates that cerPAC (200 µg mL⁻¹) impairs production of AHL-type QS molecules in wild type strains *B*. *ambifaria* HSJ1 and *C*. *violaceum.* Concentrations of (A) *N*-octanoyl-homoserine lactone (C8-HSL) and *N*-hexanoyl-homoserine lactone (C6-HSL) in *B*. *ambifaria* HSJ1, and (B) *N*-hexanoyl-homoserine lactone (C6-HSL) in *C*. *violaceum* are shown as a function of cell growth (OD₆₀₀). Results are expressed as average and standard deviations (SD) from values obtained from three replications.

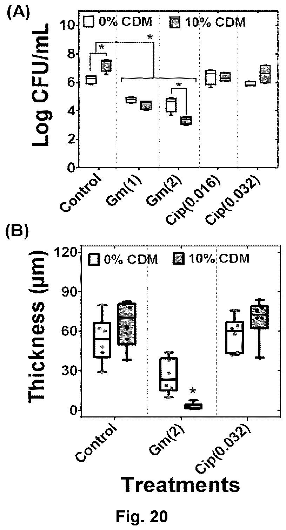

Fig. 20 illustrates the impact of antibiotics on eradication of monoculture *P. mirabilis* HI4320 biofilm at the surface of unmodified (0% CDM) and modified (10% CDM) silicone coupons. (A) Recovered bacterial cells from biofilm and (B) COMSTAT 2.1 image analysis for quantification of biofilm structures on silicone discs versus different inhibitory concentrations of gentamicin (Gm) and ciprofloxacin (Cip) for HI4320 exposed to MHB-II broth (control) or in MHB-II broth amended with individual antibiotic. Number in parentheses indicates concentration of antibiotic: 1 or 2 µg/mL gentamicin; 0.016 or 0.032 µg/mL ciprofloxacin. Error bars show the standard deviations of values obtained from four replicates. Statistically significant differences are indicated for 10% CDM-discs compared to the control (0% CDM) discs (*, *P* <0.05) and also for discs treated with antibiotic compared to discs without antibiotic (*, *P*<0.05).

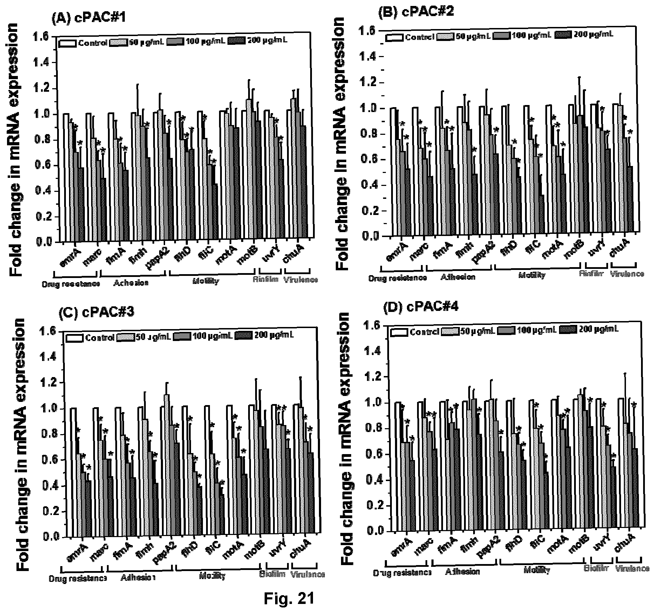

Fig. 21 illustrates the effect of cPACs on expression of drug resistance, motility, virulence and adhesion associated genes for *E*. *coli* CFT073. Error bars show the standard deviations of values obtained from three replications. All cases of gene expression were normalized to levels for the corresponding housekeeping gene gapA of *E*. *coli,* and then were related to the normalized expression level of the same gene in the control. An asterisk indicates a statistically significant difference in measured values compared to the control (*P < 0.05 by Student's t test).

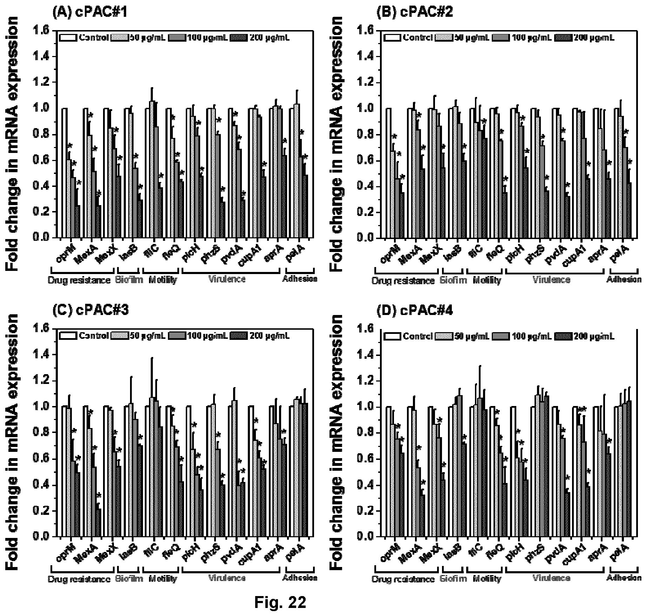

Fig. 22 illustrates the effect of cPACs on expression of drug resistance, motility, virulence and adhesion associated genes for *P. aeruginosa* PAO1. Error bars show the standard deviations of values obtained from three replicates. All cases of gene expression were normalized to levels for the corresponding housekeeping gene *rpoD* of *P. aeruginosa,* and then were related to the normalized expression level of the same gene in the control. An asterisk indicates a statistically significant difference in measured values compared to the control (*P < 0.05 by Student's t test).

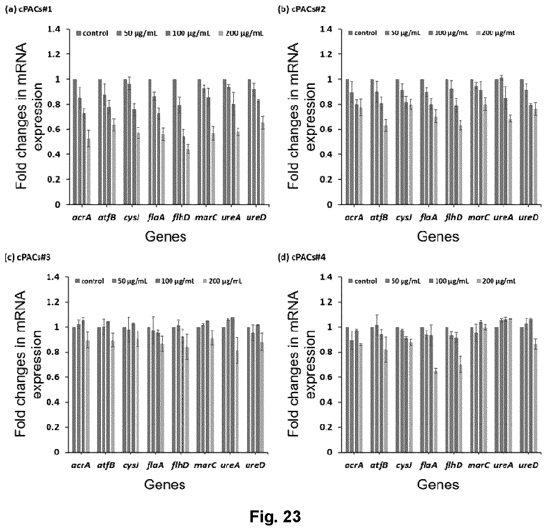

Fig. 23 illustrates the effect of cPACs on expression of drug resistance, motility, virulence and adhesion associated genes for *P. mirabilis* HI4320. Error bars show the standard deviations of values obtained from three replications of single experiment. All cases of gene expression were normalized to levels for the corresponding housekeeping genes rpoA of *P. mirabilis,* and then were related to the normalized expression level of the same gene in the control.

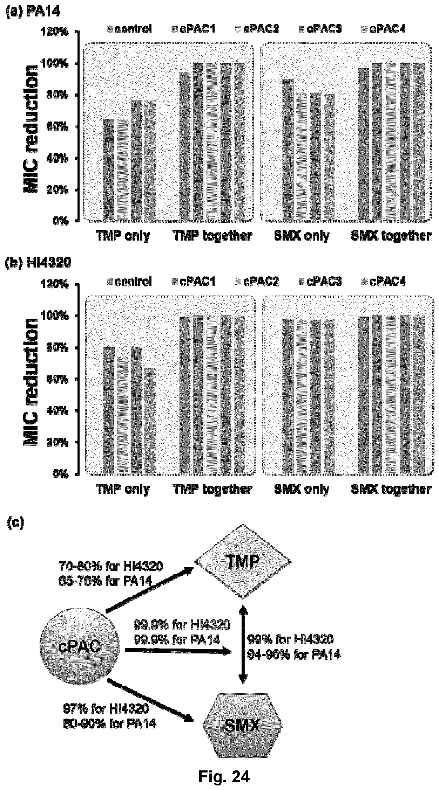

Fig. 24 illustrates that cPACs enhance synergy of the combination of trimethoprim and sulfamethoxazole antibiotics. Each cPAC reduces the MIC of trimethoprim (TMP) or sulfamethoxazole (SMX) alone or in combination (TMP+SMX) for the growth inhibition of (A) P. *aeruginosa* PA14 and (B) P. *mirabilis* HI4320. (C) Schematic showing average percentage of MIC reduction in TMP or SMX alone or TMP-SMX combination usage due to cPACs incorporation.

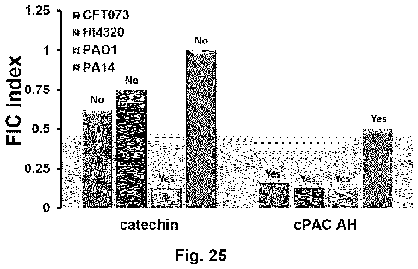

Fig. 25 illustrates the synergy of catechin or cPAC-AH (cPAC sample provided by A. Howell from Rutgers University, NJ) with trimethoprim. FIC index for each combination of TMP in combination with catechin or cPAC-AH against four pathogenic strains. Antibiotic synergy is indicated with yes or no.

### DETAILED DESCRIPTION

It is provided a synergistically active composition comprising a cranberry extract and an antibiotic for treating a bacterial infection.

Compounds derived from the American cranberry *(V. macrocarpon* L.) have been reported to exhibit anti-oxidant, anti-adhesion, anti-motility and anti-cancer activities. Herein, it is provided the anti-bacterial efficacy of the composition described herein comprising cranberry-derived proanthocyanidins and antibiotic and its potential in treating clinical and multiple drug resistant pathogenic bacterial strains.

Bacteria have evolved multiple strategies for causing infections that include producing virulence factors, undertaking motility, developing biofilms, and invading host cells. N-acylhomoserine lactone (AHL)-mediated quorum sensing (QS) tightly regulates the expression of multiple virulence factors in the opportunistic pathogenic bacterium *Pseudomonas aeruginosa.* It is demonstrated herein an anti-virulence activity of a cranberry extract rich in proanthocyanidins (cerPAC or cPAC) against *P. aeruginosa* in the model host *Drosophila melanogasterand* show this is mediated by QS interference. cerPAC reduced the production of QS-regulated virulence determinants and protected D. *melanogaster* from fatal infection by *P. aeruginosa* PA14. Quantification of AHL production using liquid chromatography-mass spectrometry confirmed that cerPAC effectively reduced the level of AHLs produced by the bacteria. Furthermore, monitoring QS signaling gene expression using *lacZ* fusion reporters revealed that AHL synthases Lasl/Rhll and QS transcriptional regulators LasR/RhIR genes were inhibited and antagonized, respectively, by cerPAC. Molecular docking studies suggest that cranberry-derived proanthocyanidin binds to QS transcriptional regulators, mainly interacting with their ligand binding sites.

The fruit of the American cranberry *(Vaccinium macrocarpon* L.) has been anecdotally reported as a natural remedy for urinary tract infections for centuries. Accordingly, a growing number of studies have examined the potential anti-oxidant, anti-adhesion, anti-motility and anti-cancer properties of cranberry-derived compounds. A number of these studies focused on the bioactivity of a specific fraction of cranberry phytochemicals known as proanthocyanidins (cPACs). Research shows that these condensed tannins hinder bacterial attachment to cellular or biomaterial surfaces, impair motility of the pathogens *Pseudomonas aeruginosa, Escherichia coli* and *Proteus mirabilis,* and can induce a state of iron limitation in uropathogenic E. *coli.* While many studies have suggested that consumption of cranberry-derived materials, namely cranberry capsules and cranberry juice, is effective in preventing bacterial infections, few have looked at the effects of these cranberry-derived materials *in vitro* and *in vivo* after consumption. Indeed, the effect of bioactive cPACs on bacterial virulence in *vivo* and mechanisms by which they do so are poorly understood. To date, not much attention has been given to the anti-virulence properties of cPACs.

*P. aeruginosa* is an opportunistic and versatile γ-proteobacterium that readily develops antibiotic resistance and is responsible for various infections affecting immunocompromised individuals, such as those suffering from cystic fibrosis. *P. aeruginosa* regulates most of its virulence factors in a cell density-dependent manner via cell-to-cell communication, commonly known as quorum sensing (QS). *P. aeruginosa* has two major *N*-acylhomoserine lactone (AHL)-based QS pathways, the Las and Rhl QS systems, and one 2-alkyl-4-(1H)-quinolone (AQ)-based QS system, which function in a cascade manner. The Las system is positioned at the top of the QS hierarchy and uses N-3-(oxo-dodecanoyl)-L-homoserine lactone (3-oxo-C₁₂-HSL) as its signal molecule, and involves Lasl and LasR as the synthase and regulator, respectively. The Rhl system uses N-butanoyl-L-homoserine lactone (C₄-HSL) as its signal, and involves Rhll and RhlR as the synthase and regulator, respectively. The LasR initiates the QS regulatory systems, partially activates the transcription of RhlR and other regulators of *Pseudomonas* quinolone signal (PQS) and integrated quorum sensing (IQS) systems. The complex QS regulation network influences, both positively and negatively, the transcription of possibly 5-10% genes of P. *aeruginosa.* The QS system is an essential part of the organism's virulence and is required to establish infection in the mammalian host.

It was reported that cranberry proanthocyanidins slightly potentiate the effect of the antibiotic activity of gentamicin (Ulrey et al., 2014, BMC Complementary and Alternative Medicine, 14: 499). On the contrary, as demonstrated herein, it is disclosed a synergistically active composition comprising a cranberry extract and an antibiotic for treating a bacterial infection.

Four different fractions of cranberry proanthocyanidins were tested, as provided by Ocean Spray Cranberries (see Table 1).

**Table 1**

| Extent of antibiotic synergy of different cPAC samples against *Escherichia coli* CFT073, *Proteus mirabilis* H14320, *Pseudomonas aeruginosa* PAO1, *Pseudomonas aeruginosa* PA14 and *Enterococcus faecalis* ATCC 29212 | | | | | | | | | | |
|---|---|---|---|---|---|---|---|---|---|---|
| | | % reduction in antibiotic usage^{b} | | | | | | | | |
| | Bacterial strains | GEN | TET | AZT | KAN | TMP | SMX | FOS | NIT | NOR |
| cPAC-1 ^{a} | CFT073 | 75 | 50 | 75 | 75 | 86 | 97 | 50 | 25 | 90 |

| | | % reduction in antibiotic usage^{b} | | | | | | | | |
|---|---|---|---|---|---|---|---|---|---|---|
| | Bacterial strains | GEN | TET | AZT | KAN | TMP | SMX | FOS | NIT | NOR |
| | PAO1 | 75 | 75 | 50 | 75 | 85 | 97 | 98 | 58 | 77 |
| | HI4320 | 78 | 85 | 96 | 88 | 81 | 97 | 97 | 94 | 25 |
| | PA14 | 63 | 66 | 96 | 97 | 70 | 90 | 84 | 91 | 72 |
| | ATCC 29212 | ND | ND | ND | ND | 82 | 58 | 74 | 75 | 81 |
| cPAC-2^{a} | CFT073 | 88 | 75 | 50 | 94 | 86 | 97 | 50 | 38 | 55 |
| | PAO1 | 88 | 88 | 75 | 50 | 58 | 97 | 98 | 71 | 81 |
| | HI4320 | 86 | 80 | 89 | 89 | 75 | 90 | 97 | 94 | 84 |
| | PA14 | 63 | 98 | 38 | 96 | 70 | 81 | 91 | 91 | 90 |
| | ATCC 29212 | ND | ND | ND | ND | 81 | 66 | 94 | 79 | 79 |
| cPAC-3^{a} | CFT073 | 88 | 88 | 75 | 88 | 81 | 95 | 91 | 69 | 63 |
| | PAO1 | 75 | 0 | 75 | 0 | 58 | 97 | 81 | 84 | 81 |
| | HI4320 | 95 | 93 | 97 | 91 | 81 | 90 | 98 | 94 | 0 |
| | PA14 | 93 | 83 | 29 | 89 | 58 | 81 | 97 | 85 | 77 |
| | ATCC 29212 | ND | ND | ND | ND | 89 | 75 | 94 | 75 | 88 |
| cPAC-4^{a} | CFT073 | 88 | 88 | 75 | 94 | 88 | 92 | 65 | 75 | 90 |
| | PAO1 | 75 | 0 | 75 | 50 | 58 | 97 | 97 | 50 | 81 |
| | HI4320 | 76 | 63 | 79 | 91 | 58 | 90 | 96 | 94 | 0 |
| | PA14 | 29 | 64 | 30 | 96 | 75 | 80 | 76 | 94 | 79 |
| | ATCC 29212 | ND | ND | ND | ND | 95 | 50 | 86 | 75 | 83 |

| | | | | | | | | | | |
|---|---|---|---|---|---|---|---|---|---|---|
| ^{a}cPAC-1, ~95%(w/w) PACs enriched from cranberry fruit juice extract; cPAC-2, ~95%(w/w) PACs enriched from cranberry extract; cPAC-3, ~95%(w/w) PACs enriched from cranberry juice; cPAC-4, 57%(w/w) PACs enriched polyphenolic extract containing flavonols and anthocyanins. ^{b}ND: not determined; GEN: gentamicin; KAN: kanamycin; TET: tetracycline; AZT: azithromycin; TMP: trimethoprim; SMX: sulfamethoxazole; FOS: fosfomycin; NIT: nitrofurantoin; NOR: norfloxacin. | | | | | | | | | | |

To verify the antibiotic synergy of cranberry proanthocyanidins (cPACs) with targeted types of antibiotics of interest in the context of urinary tract infections, checkerboard assay was performed for *Escherichia coli* CFT073, *Proteus mirabilis* H14320, *Pseudomonas aeruginosa* PAO1, *P. aeruginosa* PA14 and *Enterococcus faecalis* ATCC 29212. Trimethoprim, sulfamethoxazole, fosfomycin, nitrofurantoin, norfloxacin antibiotics are commonly used for the treatment of urinary tract infections. Antibiotic interaction was analyzed for the combinations of each antibiotic with cPAC sample. Fig. 1 shows the fractional inhibitory concentration (FIC) index values of each cPAC fraction in the presence of different types of antibiotics for five pathogenic strains. The corresponding FIC index values of the combination of sulfamethoxazole with all four cPAC samples were < 0.5 for five pathogenic bacterial strains, demonstrating a synergistic effect (Table 1).

In order to verify whether antibiotic synergy of cPAC can limit infection *in vivo,* a fruit fly killing assay was used in which cPAC or kanamycin alone or in combination were administered to *Drosophila melanogaster* flies infected with P. *aeruginosa* PA14. As shown in Fig. 1F, the median survival of *D*. *melanogaster* after exposure to *P. aeruginosa* was 168 h without cPAC (PA14 only), but 268 h with cPAC-Kan combination treatment, which is significantly (χ2 = 17.19, df = 5, P<0.01) less virulence based on the comparison of survival curves. The median survival of infected *D*. *melanogaster* with cPAC only and Kan alone was 240 h and 192 h, respectively. The survival of uninfected *D*. *melanogasterwas* identical to the treatment with only cPAC (without PA14).

To further investigate the mechanism of action behind the synergy between tetracycline and cPACs, tetracycline membrane transport assay was performed. As shown in Fig. 2A, each cPAC sample enhanced the uptake of tetracycline in E. *coli* CFT073 cells at different levels. cPAC-1 and cPAC-2 enhanced the uptake of tetracycline in *P. aeruginosa* PAO1 cells in a dose dependent manner, while cPAC-3 fails to enhance the tetracycline uptake and cPAC-4 support at lower level compare to cPAC-1 and cPAC-2 (Fig. 2B). Results shown in Figs. 2C and 2D show the uptake of tetracycline in *P. mirabilis* HI4320 and *P. aeruginosa* PA14 in absence and presence of cPAC samples at different concentrations. The sample cPAC-3 enhances tetracycline uptake in a dose dependent manner compared to cPAC-1, -2 and -4 for HI4320, while cPAC-3 fails to enhance the tetracycline uptake in PA14 cells compared to cPAC-1, -2 and -4. This mechanism of action correlates with the measured synergy of each cPAC sample (see Table 1) with tetracycline for each strain.

Growth curve measurements show that each cranberry proanthocyanidin fraction (without antibiotic) did not reduce the growth rates of *E. coli* CFT073 and *P. aeruginosa* PAO1 when compared to untreated cells of each strain (Fig. 3). This demonstrates that the observed bioactivity of the cranberry proanthocyanidins extract is not a killing effect but rather a synergism with the antibiotic.

Cranberry proanthocyanidins also significantly reduced biofilm formation formed by *E. coli* CFT073 at sub-lethal concentrations (see Fig. 4). Proanthocyanidins derived from cranberry cause cell membrane permeabilization and efflux pump inhibition of pathogenic bacteria without affecting cell membrane integrity.

To analyze anti-biofilm activity of cPAC for *E*. *coli* CFT073, *P. mirabilis* HI4320 and *P. aeruginosa* PA14, sub-lethal concentrations of cPAC (50 and 100 ug/mL) without and with gentamicin were chosen for biofilm studies. As shown in Fig. 4 and 5, cPAC in combination with gentamicin (at sub-lethal concentrations) had significant inhibitory effects on biofilm formation (P<0.05) for CFT073 and HI4320 in dose dependent manner. In case of PA14, cPAC in combination with gentamicin (at sublethal concentrations) failed to inhibit biofilm formation (Fig. 6). This indicates that cPAC fractions in combination with gentamicin at sub-lethal concentration are effective to inhibit biofilm formation of CFT073, PAO1 and HI4320.

The specific mechanism(s) of action for the observed synergistic interactions between proanthocyanidins and antibiotic is disclosed. As mentioned herein above, the proanthocyanidins at sub-inhibitory concentrations permeabilize the cell outer-membrane and inhibit multidrug resistance efflux pumps involved in multidrug resistance in pathogenic bacteria, without affecting cell membrane integrity (see Figs. 7-9). This is interesting, because elimination of persister cells at sub-inhibitory concentrations of cranberry proanthocyadins can reduce the amount of antibiotic required for the treatment of chronic and recurrent infections. The beneficial properties of cranberry proanthocyanidins suggest that the combination of the natural compounds and antibiotics is an effective new anti-bacterial therapy.

Treatment with cerPAC significantly inhibited the staphylolytic (LasA, *F_{3,8}=* 21.41, *p* <0.001), elastolytic (LasB, *F_{3,8}=* 84.29, *p* <0.001) and alkaline proteolytic (AprA, *F_{3,8}=* 34.41, *p* <0.001) activities of *P. aeruginosa* PA14 (Fig. 10A). Importantly, this inhibition was achieved without affecting bacterial growth (Fig. 10B).

To verify whether cerPAC can limit infection *in vivo,* a fruit fly killing assay was used in which cerPAC was administered to *Drosophila melanogaster* infected with WT *P. aeruginosa* PA14. As shown in Figure 11, the median survival of *D*. *melanogaster* after exposure to *P. aeruginosa* was 168 h without cerPAC, but 240 h with cerPAC treatment, which is significantly (χ² = 4.14, *df=* 1, *P* <0.05) less virulence based on the comparison of survival curves. The survival of uninfected *D*. *melanogaster was* identical to the treatment with only cerPAC.

The difference in the treated or untreated PA14 strains' ability to kill *D*. *melanogaster* in this feeding assay may have been due to modified survival of the bacteria on the filter papers used for exposure during incubation. To address this possibility, the survival of PA14 was analyzed on the paper discs without and with 200 µg mL⁻¹ cerPAC under the same conditions as the fly feeding assay. There was no significant difference (*F_{5,30}*= 0.54, *P=* 0.74) in culturability of the bacterium on the filter paper discs in the absence and presence of cerPAC during incubation (see Fig. 12), indicating that an alteration in survival ability of bacteria could not account for the observed differences in fly killing. Overall, these results indicate that cerPAC protect D. *melanogaster from P. aeruginosa* infection.

To determine the ability of cerPAC to modulate the production of the two principal AHL molecules by *P. aeruginosa* PA14, AHL production kinetics were determined in absence or presence of 200 µg mL⁻¹ cerPAC. As shown in Figures 13A and 13B, cerPAC significantly impairs the production of 3-oxo-C₁₂-HSL (*t*= 7.45, *df*= 4, *p* <0.001) and C₄-HSL (*t*= 3.54, *df*= 4, *p* <0.05), in *P*. *aeruginosa* PA14 at exponential and late stationary phase, respectively. This reduction in the production of the QS signals was observed without affecting bacterial growth (Fig. 13C).

To understand the mechanism for the reduction in AHL levels, *β-*galactosidase transcriptional fusion reporters of *lasl* (3-oxo-C₁₂-HSL synthase) and *rhll* (C₄-HSL synthase) were assayed in *P. aeruginosa* PA14 bioreporter strain with the same 200 µg mL⁻¹ cerPAC exposure. These bioassays revealed that expression of both AHL synthase genes (*lasI* and *rhlI*) is repressed by cerPAC (Figs. 14A and B). Similarly, it was investigated whether presence of cerPAC affects the expression of the two cognate transcriptional regulator genes *lasR* and *rhlR* using *lacZ* transcriptional fusion reporters. Expression of both regulator gene fusions was partially repressed in the presence of cerPAC (Figs. 14C and D). Thus, cerPAC inhibits both AHL synthases and partially represses the LuxR-type regulator genes associated with the production of the two AHL signals in *P. aeruginosa* PA14.

It was further investigated whether cerPAC affects LasR and/or RhlR induction by exogenous AHLs using bioreporter AHL-negative PA14 mutants with IacZfusions. As expected, when 3-oxo-C₁₂-AHL or C₄-HSL were supplied to their respective bioreporters, they activated the expression of *lasl* and *rhll,* respectively (Figs. 15A, B). While cerPAC had no effect on the activity of the reporters, there was a significant (*p* <0.05) reduced activation by either AHLs in presence of cerPAC (Figs. 15A, B). This indicates that cerPAC partially inhibits the activation of both LasR- and RhlR-directed transcription of *lasI* and *rhlI,* respectively, the primary targets of these LuxR-type regulators. Additionally, LasR and RhlR activation titration was performed in absence and presence of three different concentrations of cerPAC, which resulted in lower activation of LasR and RhlR (Figs. 15C, D). This indicates that cerPAC can reduce the activation of both regulators by their native AHLs, likely as a potential antagonist.

To assess a possible physical interaction between cerPAC components and either AHL molecule, C₄-HSL and 3-oxo-C₁₂-HSL were quantified in cell-free growth medium using an ethyl acetate extraction procedure followed by LC-MS analysis. As shown in Fig. 16, there was no difference in the concentration of AHLs with or without cerPAC, demonstrating that cerPAC components do not bind to the AHLs and therefore do not inhibit QS by physical interaction.

Inhibition of Las-type QS regulators' activities by cerPAC may be due to structural interactions, important for the functional activity of transcriptional regulatory proteins. To address this possibility, *in silico* docking analysis was performed using protein structures of LasR (2UV0 (Bottomley et al., 2007, J Biol Chem, 282: 13592-13600)), Lasl (1RO5 (Gould et al., 2004, Mol Microbiol, 53: 1135-1146)), the monomer and dimer of epicatechin molecules (important components of cPACs). The interaction energy scores (obtained using Moldock tools) of the predicted docking complex and the known crystallographic complex structures of the LasR with ligand 3-oxo-C₁₂-HSL were compared. The Moldock interaction energy score of -144.1 kcal mol⁻¹ for the predicted complex of LasR with 3-oxo-C₁₂-HSL was marginally lower than the Moldock interaction energy score of -157.5 kcal mol⁻¹ obtained for the crystallographic complex of LasR with ligand 3-oxo-C₁₂-HSL (Fig. 17A and Table 2).

**Table 2**

| Interaction energies of ligand-protein complexes during *in silico* docking analysis for Las regulatory proteins | | | | | | | | |
|---|---|---|---|---|---|---|---|---|
| | **Interaction energy scores (kcal mol⁻¹)^{d} for proteins** | | | | | | | |
| | **LasR^{c}** | | | | **LasI^{c}** | | | |
| **Ligands** | ***Eᵢₙₜᵣₐ*** | **TS** | ***Eᵢₙₜₑᵣ*** | **Moldock** | ***Eᵢₙₜᵣₐ*** | **TS** | ***Eᵢₙₜₑᵣ*** | **Moldock** |
| 3-oxo-C12-HSL(C)^{a} | -8 | 10.4 | -160.4 | -157.5 | NA | NA | NA | NA |
| 3-oxo-C12-HSL(P)^{b} | -12 | 24.3 | -156.6 | -144.1 | NA | NA | NA | NA |
| S-adenosyl L methionine | NA | NA | NA | NA | -29.7 | 7 | -103.6 | -126.2 |
| Epichatechin | 14.7 | 0.2 | -142 | -127.1 | 12.1 | 0.2 | -118.8 | -106.8 |
| Proanthocyanidin | 80.5 | 2 | -150.3 | -68 | 15.6 | 1.3 | -170.5 | -153.6 |

| | | | | | | | | |
|---|---|---|---|---|---|---|---|---|
| ^{a}C, structural pose during crystallographic analysis reported by Bottomley et al. (2007, J Biol Chem., 282: 13592-13600) ^{b}P, predicted structural pose during our docking analysis *^{c}Eᵢₙₜᵣₐ,* internal ligand energy between atoms of the ligand (electrostatic, steric and hydrogen interactions); *Eᵢₙₜₑᵣ,* ligand-protein interaction energy; TS, energy penalties for the internal torsional strain of a ligand; Moldock, total interaction energy using Moldock scoring function. ^{d}NA, not available | | | | | | | | |

The epicatechin and its dimer (proanthocyanidin) molecules were docked separately in the internal cavity of LasR (Figs. 17B and C). Ligand binding domain (LBD) of LasR with a volume of 653 Å³, exhibits sufficient space to accommodate the monomer or dimer of epicatechin with a volume of 225 Å³ or 466 Å³, respectively. The *in silico* docking analysis suggests that the complex formation between the epicatechin and LasR, with a Moldock interaction energy score of -127.1 kcal mol⁻¹, is more favorable than LasR-proanthocyanidin complex with Moldock score of -68 kcal mol⁻¹ (Table 2). The proanthocyanidin formed six hydrogen bonds at the internal binding cavity of LasR compared to four hydrogen bonds of the LasR-3-oxo-C₁₂-HSL or LasR-epicatechin complex (Fig. 17). The increase in the Moldock score for the docking complex of LasR with proanthocyanidin compared to LasR-epicatechin complex was observed due to the steric constraints of the proanthocyanidin structure in the internal cavity space of LasR identified by the comparison of their internal energies (Table 2).

Due to the lack of crystallographic structure of Lasl protein bound with its natural substrates or functional analogues, an *in silico* docking analysis was performed to predict a complex of Lasl with its natural substrate S-adenosyl L methionine (SAM) (Fig. 18A). This putative complex with Lasl was used as a reference for both docking analyses of epicatechin and proanthocyanidin. The best five structural positions of SAM with higher interaction energies occupied the same binding cavity on the Lasl protein. The docking analysis showed the formation of hydrogen bonds of SAM with residues that surround the putative binding cavity with Moldock interaction energy score of -126.2 kcal mol⁻¹ (Fig. 18A and Table 2). The binding cavity known for the second substrate of Lasl, the acyl-acyl carrier protein (acyl-ACP) was not identified as a potential binding site for either of the tested cerPAC components (epicatechin or proanthocyanidin). The Lasl-epicatechin complex showed single hydrogen bond with Moldock interaction energy score of -106.8 kcal mol⁻¹ (Fig. 18B and Table 2). The docking complex of the Lasl protein with the proanthocyanidin molecule suggests the more favorable complex formation with five hydrogen bonds and Moldock interaction energy score of -153.6 kcal mol⁻¹ compared to the Lasl-SAM complex (Fig. 18C). This *in silico* docking analysis suggests that both main components of cerPAC have the potential to form complexes with LasR and Lasl proteins to compete with their native ligands 3-oxo-C₁₂-HSL and SAM, respectively.

As demonstrating herein, cerPAC acts as a general QS inhibitor by interfering with the binding of the AHL ligand to LuxR-type transcriptional regulators. To verify that cerPAC is able to impede QS in other bacterial species, an AHL production kinetics assay was performed to examine the effect of administering cerPAC to wild type strains of *Burkholderia ambifaria* and *Chromobacterium violaceum.* The addition of cerPAC to growth medium significantly impairs the production of the two main AHLs (Cs-HSL and C₆-HSL) in *B*. *ambifaria* (Fig. 19A) and C₆-HSL in C. *violaceum* (Fig. 19B). Since the primary target of LuxR regulators are *luxl* homologues, these observations validate the capacity of cerPAC to interfere with AHL-mediated QS in different bacterial species.

It is showed herein that a cranberry extract enriched in PACs restricts virulence of *P*. *aeruginosa* in a fruit fly animal model and inhibits QS mechanisms. In addition, the cerPAC does not perturb cell viability of *P. aeruginosa,* indicating that use of these molecules may provide less selective pressure towards the development of resistance than conventional antibiotics (bactericidal and bacteriostatic, which pose strong selective pressure in any environment). The anti-virulence efficacy of cerPAC is based on: 1) it reduces the production of AHL signaling molecules; 2) it represses the expression of the QS regulators LasR and RhlR and autoinducer synthases Lasl and Rhll; 3) it antagonizes the activation of LasR and RhlR by their cognate autoinducers; and 4) epicatechin and proanthocyanidin, the main components of cerPAC, are modeled *in silico* to interact with the LBD of LasR and LasI. In addition, cerPAC also inhibit AHL production in strains of the Gram-negative species B. *ambifaria* and C. *violaceum.* Thus, cerPAC could have anti-virulence activity against various pathogens with clinical importance.

For *in vivo* study, a fly feeding assay was used because it represents a long-term infection model and involves feeding starved flies with bacterial cultures. This method is better adapted to chronic infections compared to the fly nicking model. A dose of cerPAC was supplied at the start of infection and virulence was subsequently reduced, indicating that a cranberry extract enriched in PACs could function as a prophylactic. These results, when considered with other literature, indicate that the use of effective prophylactic molecules with anti-virulence activity, specifically for *P. aeruginosa,* could be a best practice in the clinical setting. It is noteworthy that the extract used herein contains approximately 95% proanthocyanidins, and thus, it is presumed that the bioactivity observed can be mostly attributed to these molecules.

That a cerPAC alone inhibits QS has not been previously reported in the peer-reviewed scientific literature. Las and Rhl OS systems were targeted because they are at the top of the *P. aeruginosa* quorum sensing hierarchy. Both AHL molecules induce their own production and activate the corresponding LuxR-type transcriptional regulators LasR and RhlR. In the presence of the cranberry extract, an impairment in AHL production was observed, along with reduced gene expression of AHL synthase (LasI and Rhll) and partial repression of their regulators (LasR and RhIR). Interestingly, it is demonstrated that cerPAC, a potent *in vivo* inhibitor, is an effective antagonist of both LasR and RhIR, two regulators that act reciprocally on key virulence determinants.

The successful molecular docking of epicatechin or proanthocyanidin with LasR demonstrates that the inactivation of transcriptional regulators may be the primary mechanism of action for the cerPAC as anti-virulence factors *in vivo.*

The results disclosed herein show that cerPAC protects D. *melanogaster* from *P. aeruginosa* likely through an inhibition of QS without negative effect on bacterial growth. Antagonist activity and *in silico* analysis projected the potential mechanism of action to the inhibition of AHL regulators.

It was also demonstrated that the incorporation of cranberry derived materials (CDM) in silicone increased susceptibility of *P. mirabilis* biofilms to gentamicin, which results in biofilm disruption (see Fig. 20).

To explore the genetic basis for the synergy in antimicrobial activity observed between cPACs and antibiotics, as well as the effect of cPACs on bacterial biofilm forming potential, transcriptional analysis was performed using qRT-PCR to observe the differential expression of genes associated with multidrug resistance, bacterial motility, virulence, adhesion, and biofilm formation for each of the two bacterial strains. Gene expression of E. *coli* CFT073, P. *aeruginosa* PAO1 and *P. mirabilis* HI4320 shown in Fig. 21, 22 and 23 respectively, indicate that cPACs, at sub-lethal concentrations, repressed the expression of genes associated with multiple drug resistance *(emrA, acrB,* and *marC* in CFT073; *acrA* and *marC* in HI4320; *oprM, mexA,* and *mexX* in PAO1), motility (*fliC, flhD, motB, fimH, fimA,* and *papA2* in CFT073; *flaA* and *flhD* in HI4320; *fliC* and fleQ in PAO1), virulence determinants (*chuA* in CFT073; *cysJ* in HI4320; *plcH, phzS,* and *pvdA* in PAO1), adhesion (*fimH, fimA,* and *papA2* in CFT073; *atfB* in HI4320; *cupA1* and *pelA* in PAO1), and biofilm formation (*uvrY* in CFT073; *ureD* in HI4320; *lasB* in PAO1). This transcriptional analysis confirms the trends observed with the antibiotic synergy and biofilm assays.

The sulfamethoxazole (SMX) antibiotic is known to synergize with trimethoprim (TMP) and they are commonly used in combination for clinical applications. To investigate the interaction between cPAC fraction and the combination of the two antibiotics, a checkerboard assay was performed with mixture of TMP-SMX at sub-inhibitory concentration ratio with each cPAC to analyze growth inhibition of HI4320 and PA14. The cPAC enhanced the synergy of TMP-SMX combination and reduced MIC up to 99.9%, which is higher compared to the measured synergy of cPAC with individual antibiotics TMP and SMX for the growth inhibition of PA14(Fig. 24A-C).

There is a possibility that catechin (a monomer of cPAC structure) and cPACs from different sources could synergize differently with antibiotics compared to the cPAC fractions tested above. To investigate this possibility, the checkerboard assay was performed using four bacterial strains and analyzed for determination of FIC index values. As shown in Fig. 25, catechin synergizes with trimethoprim for the growth inhibition of *P. aeruginosa* PAO1 and fails to show synergy for the other three strains, while a cPAC sample obtained from Dr. Amy Howell (Rutgers University) (cPAC-AH) synergizes with trimethoprim for the growth inhibition of all four strains based on FIC index ≤0.5.

Cranberry powder from dehydrated whole crushed cranberries (Atoka Cranberries, Quebec, Canada) was used as the cranberry derived material (CDM) of interest. CDM was incorporated into LSR30 implant grade silicone. First, *P. mirabilis* biofilms were grown on CDM-modified silicone surface and subsequently treated with gentamicin and ciprofloxacin at sub-MICs. A synergy of CDMs with gentamicin was observed.

Encompassed herein is the combination of the cranberry extract and composition described herein with an antibiotic according to claim 1..

Also encompassed is the combination of the cranberry extract and composition described herein with different materials used in the art for non-limiting application in medical settings such as natural anti-infective, antimicrobial, anti-biofilm or anti-virulence agent in individual or combinatorial therapies thereof.

Further encompassed is the combination of the cranberry extract and composition described herein with materials used for non-limiting applications such as edible or non-edible functional or non-functional food coatings or food packaging
The present disclosure will be more readily understood by referring to the following examples.

### EXAMPLE I

### Minimum Inhibitory Concentration (MIC)

Five organisms were used to demonstrate the efficacy of the composition described herein: *E. coli* strain CFT073 (ATCC 700928), *P. mirabilis* HI4320 (Mobley and Warren, 1987, J Clin Microbiol, 25: 2216-2217), *P. aeruginosa* PAO1 (ATCC 15692), *and P. aeruginosa* PA14 (UCBPP-PA14, Rahme et al., 1995, Science, 268: 1899-1902*), P. aeruginosa* PAO1 (ATCC 15692) and *E*. *faecalis* ATCC 29212. Pure stock cultures were maintained at - 80°C in 30% (v/v) frozen glycerol solution. Starter cultures were prepared by streaking frozen cultures onto LB agar (LB broth: tryptone 10 g/L, yeast extract 5 g/L and NaCl 5 g/L, supplemented with 1.5 w/v % agar (Fisher Scientific, Canada)). After overnight incubation at 37°C, a single colony was inoculated into 10 mL of LB broth and the culture was incubated at 37°C on an orbital shaker at 150 rpm for time lengths specific to each experiment. LB broth was used for bacterial culture in all experiments unless otherwise specified.

Minimum Inhibitory Concentration (MIC) was determined by preparing two-fold serial dilutions of each cPACs fraction and antibiotic in Mueller Hinton broth adjusted with Ca²⁺ and Mg²⁺ (MHB-II, Oxoid, Fisher Scientific, Canada). A range of concentration of the antibiotics gentamicin (0.0156-2 µg/mL), tetracycline (0.03-4 µg/mL), kanamycin (0.25-512 µg/mL), azithromycin (0.125-256 µg/mL), trimethoprim (0.25-512 µg/mL), sulfamethoxazole (0.25-512 µg/mL), nitrofurantoin (0.25-512 µg/mL), fosfomycin (0.25-512 µg/mL), norfloxacin (0.031-2 µg/mL), ciprofloxacin (0.0003-1 µg/mL) and ampicillin (0.25-2000 µg/mL), was used. Dilutions were prepared in flat bottom, 96 well microtitre plates (untreated, Falcon, Corning, Fisher Scientific, Canada). Each well of a microtitre plate was then inoculated with the desired bacterial strain (grown in MHB-II and diluted to 10⁶ CFU/mL) and the plate was incubated at 37°C for 18 hours under static conditions. Bacterial growth was assessed by (i) monitoring the optical density of the cell suspension in each well at 600 nm (OD600 nm), and (ii) the resazurin microtitre plate assay. In the resazurin microtitre plate assay, each well of a microtitre plate was supplemented with 20 µM resazurin, incubated in dark for 20 min at room temperature, followed by fluorescence measurements at ex/em 570/590 nm using a TECAN Infinite M200 Pro microplate reader (Tecan Group Ltd., Switzerland). The lowest concentration of a compound able to prevent increase in OD600 nm and resazurin fluorescence intensity was recorded as the MIC for that compound.

### EXAMPLE II

### Checkerboard microdilution assay

The checkerboard microdilution assay was used for evaluation of *in vitro* antimicrobial synergy between two compounds (i.e., antibiotic and each cPAC fraction). Two-fold serial dilutions were prepared in MHB-II for each of the two compounds under study. The serial dilutions were then loaded into 96 well plates to achieve combinations having different concentrations of each of the two compounds. Each well was subsequently inoculated with 10⁶ CFU/mL of the desired bacterial strain and incubated at 37 °C for 18 hours under static conditions. The Fractional Inhibitory Concentration Index (FICI) for each combination was calculated by using the following formulae: FICcomponent 1 = MICcomponent1, in combination / MICcomponent1 ,alone FICI = FICcomponent 1 + FICcomponent 2

The FICls were interpreted as follows: FICI of ≤0.5 (synergy); 0.5 <FICI ≤ 4 (no interaction/indifference); FICI of >4 (antagonism).

### EXAMPLE III

### Biofilm formation

Biofilm formation was quantified using the standard microtitre plate model. Briefly, overnight cultures (MHB-II broth, 37°C, 200 rpm) were diluted 1:100 (v/v) into fresh MHB-II broth (with or without each cPAC fraction and their combination with gentamicin), to 10⁶ CFU/mL. Aliquots (100 µL) of these cultures were transferred into the wells of polystyrene, flat bottom, non-treated 96 well plates (Falcon, Corning), in triplicate. For all assays, biofilms were allowed to develop for 18 hours at 37°C under static conditions, after which OD600 values were recorded, the spent broth was decanted from the wells and the wells were gently rinsed three times with DI water. The washed biofilm was stained with crystal violet (CV). For CV stain assay, 100 µL of 0.1% (w/v) CV was loaded in each well and the plates were incubated for 15 minutes under static condition at room temperature. The wells were subsequently rinsed with DI water to remove excess dye and the CV adsorbed to the biomass in each well was solubilized in 100 µL of absolute ethanol for 10 minutes. The solubilized CV was then quantified (as OD570) using a microplate reader. Control experiments were performed with cell-free broth to adjust for background signal.

### EXAMPLE IV

### Membrane permeabilization and membrane integrity assays

The outer membrane permeabilization activities of each cPAC fraction and antibiotic were determined by the 1-N-phenylnapthylamine (NPN, Sigma-Aldrich Canada) assay with some modifications. Briefly, overnight bacterial cultures were diluted 1:1 in MHB-II medium to a final volume of 10 mL, with or without sub-MIC supplementation of each cPACs fraction or gentamycin (as a positive control), and grown to an OD600 of 0.5-0.6 (37 °C, 200 rpm). The cells were harvested, washed with 5 mM HEPES buffer (pH 7.2), and resuspended in the same volume (10 mL) of 5 mM HEPES buffer (pH 7.2) containing 1 mM N-ethylmaleimide (NEM, Sigma-Aldrich Canada). Aliquots (1 mL) were mixed with NPN to a final concentration of 10 µM (in cell suspension) and fluorescence was measured using the microplate reader (ex/em 350/420 nm).

The BacLight kit (L-13152, Invitrogen, Life Technologies Inc., Canada) was used to assess cell membrane damage. Overnight bacterial cultures were diluted 1:40 in fresh MHB-II broth to a final volume of 5 mL, grown to an OD600 of 0.5-0.6, washed with filter-sterilized 10 mM phosphate buffered saline (PBS, pH 7.0) and resuspended in 1/10 of the original volume. The washed cells were then diluted 1:20 v/v into stock solution of each cPACs fraction at 1/2 MICs or DI water (control). Cultures were incubated at room temperature (27±2°C) on a tube rocker for 10 minutes. At the end of the incubation period, an aliquot was taken for CFU counts and the remaining suspension was washed with 10 mM PBS and resuspended to an OD600 of 0.3. An aliquot (100 µL) of each bacterial suspension was removed and added to a 96-well, black, clear-bottom plate (Corning, Fisher Scientific, ON, Canada) along with an equal volume of the BacLight reagent (2× stock solution, L13152, Invitrogen, Life Technologies Inc., Canada) and the plates were incubated for 10 minutes at room temperature in the dark. At the end of the incubation period, fluorescence intensity was recorded for both kit components, SYTO-9 (ex/em 485/530nm) and propidium iodide (ex/em 485/645nm), using the microplate reader. Fluorescence readings from samples were normalized to the values obtained from untreated control to determine the ratio of membrane compromised cells to cells with intact membrane. CTAB (Sigma-Aldrich Canada), a cationic detergent that is known to cause membrane damage, was used at 1/2 MICs as a positive control for membrane disruption.

### EXAMPLE V

### Ethidium bromide (EtBr) efflux assay

To assess the effect of each cPAC fraction on the inhibition of the proton motive force driven multidrug efflux pump, an ethidium bromide (EtBr) efflux assay was performed. An overnight grown culture of each strain was diluted 1:100 using MHB-II broth to a final volume of 10 mL and grown to an OD600 of 0.8-1.0 (at 37 °C, 150 rpm). Cells were loaded in polystyrene microcentrifuge tubes (2 mL) and mixed with 5 µM EtBr and each cPAC fraction at 25% of their MIC, or 100 µM of the proton conductor, carbonyl cyanide m-chlorophenylhydrazone (CCCP, Sigma-Aldrich Canada), as positive control. Replica tubes that did not receive cPAC or proton conductor served as negative controls. The tubes were incubated for 1 hour (37°C, 150 rpm). The inoculum was then adjusted to 0.4 OD600 with MHB-II broth containing 5 µM EtBr and 2 mL aliquots of this mixture were pelleted (5000×g, 10 min at 4°C). The pellets were incubated on ice immediately, resuspended in 1 mL of MHB-II and aliquoted (200 µL) into a polystyrene 96 well, black, clear-bottom plate (Corning, Fisher Scientific, Canada). EtBr efflux from the cells was monitored at room temperature using the microplate reader (ex/em 530/600 nm). Readings were taken at 5 minute intervals for 1 hour to monitor efflux pump activity. The background fluorescence of the medium was subtracted from all measurements and the assay was repeated independently in triplicate.

### EXAMPLE VI

### Cranberry-derived materials and bacterial strains

The cranberry extract rich in proanthocyanidins (cerPAC) was obtained from Ocean Spray Cranberries Inc. The supplier prepared the sample according to well established methods by enriching from cranberry fruit juice extract. The exact composition contains approximately 95% proanthocyanidins. A dry powder of cerPAC was solubilized in deionized water and sterilized by filtration (0.22 µm PVDF membrane filter). Bacteria used in this study were *P. aeruginosa* strain PA14 (wild type) and isogenic QS mutant strains in *lasI, rhlI, lasR* and *rhlR* as well as wild type strains *Burkholderia ambifaria* HSJ1, *Chromobacterium violaceum* ATCC 31532 and *Staphylococcus aureus* ATCC 25923. Plasmids carrying *lacZ* fusion with genes *lasI* (pSC11, transcriptional fusion; pME3853, translational fusion), *lasR* (pPCS1001, transcriptional fusion), *rhlI* (pMW305, transcriptional fusion; pME3846, translational fusion) and *rhlR* (pPCS1002, transcriptional fusion) were introduced into appropriate *P. aeruginosa* PA14 QS mutant strains by electroporation, as described previously. All bacterial strains were preserved in glycerol stock (15% v/v) culture at -80°C and cultured in Tryptone Soy Broth (TSB) medium, with antibiotics if required for plasmid maintenance: tetracycline (75 mg L⁻¹), carbenicillin (300 mg L⁻¹), gentamicin (100 mg L⁻¹), streptomycin (250 mg L⁻¹) and spectinomycin (250 mg L⁻¹).

### EXAMPLE VII

### Phenotypic assay

To assess LasB elastolytic activity, filter-sterilized culture supernatant samples (100 µL) from late stationary phase cultures of strain PA14 were mixed with 5 mg elastin Congo red reagent (Sigma-Aldrich) and 300 µL 0.1 M Tris-HCl (pH 7.2). Release of Congo red from degraded elastin was measured as A₄₉₅ after 2 h of incubation at 37°C with shaking at 100 rpm, followed by centrifugation. For assessment of LasA staphylolytic activity, 5 mLof S. *aureus* ATCC 25923 overnight cultures were boiled for 15 min, and 100 µl were mixed with 300 µL of filtered culture supernatants of PA14. The OD₆₀₀ was measured after 2 h of incubation at 37 °C and 100 rpm. To analyze alkaline protease (AprA) activity, filter-sterilized culture supernatant samples (200 µL) from late stationary phase cultures of PA14 were vortexed with 25 mg of Hide-Remazol Brilliant Blue R powder (Sigma-Aldrich) in 800 µL of 20 mM Tris-HCl buffer (pH 8.0) containing 1 mM CaCl₂. The tube was then incubated at 37°C at 150 rpm for 1 h. The insoluble hide azure blue was removed by centrifugation at 10,000 × g for 4 min at 4°C and the absorption of the supernatant was measured at 595 nm. All experiments were carried out in triplicate.

### EXAMPLE VIII

### Infection of Drosophila melanogaster

Fruit flies (*D. melanogaster*) were infected orally in fly feeding assay as before (Lutter et al., 2008, Infect Immun, 76: 1877-1888; Apidianakis and Rahme, 2009, Nat Protoc, 4: 1285-1294), with some modifications. Briefly, flies were anesthetized under a gentle stream of carbon dioxide. Male flies (3- to 5-days-old) were starved of food and water for 5-6 h and separated into vials (10 per vial) containing 5 ml of 5% sucrose agar (sterile) without and with 200 µg mL⁻¹ cerPAC and 2.3-cm filter paper disks (sterile) containing freshly grown bacterial culture suspension. To achieve this freshly grown culture, an overnight PA14 culture was inoculated in 6 mL TSB culture and incubated at 37°C and 100 rpm until OD₆₀₀ = 3.0. This culture was centrifuged at 12,000 × g for 1 min and the resulting pellet resuspended in 150 µL of sterile 5% sucrose, without and with 200 µg mL⁻¹ cerPAC. All filters were soaked appropriately with this culture suspension, along with sucrose agar, in feeding vials prior to transferring flies into the vial. Separate feeding vials soaked with 150 µL of 5% sucrose without and with 200 µg mL⁻¹ cerPAC were used as negative controls for each experiment. Post-infection mortality of flies was monitored daily for 14 days, with each treatment tested twice in triplicate.

### EXAMPLE IX

### LC-MS analyses

Specific estimation of AHL molecules was achieved by LC-MS in the positive electrospray ionization (ESI+) mode, combined with the MRM mode, as described previously (Lepine and Deziel, 20111, Methods Mol Biol, 692: 61-69).

Samples of PA14 culture exposed to cerPAC were retrieved at different time points and OD₆₀₀ was measured. An aliquot of methanolic internal standard was mixed with each sample to adjust final concentration 3 mg L⁻¹ of 5,6,7,8-tetradeutero-4-hydroxy-2-heptylquinoline (HHQ-d₄) and 6 mg L⁻¹ of 5,6,7,8-tetradeutero-3,4-dihydroxy-2-heptylquinoline (PQS-d₄). All culture samples were vortex-mixed and extracted twice with ethyl acetate (1:1, vol:vol), each ethyl acetate extract pooled and evaporated at 30°C under a gentle stream of nitrogen. The residue was then resuspended in acidified acetonitrile (HPLC grade, containing 1% ACS grade acetic acid) at ten times the initial concentration and 20 µl aliquots were injected for LC-MS analysis.

The LC-MS analyses were performed with a Quattro II (Waters) triple quadrupole mass spectrometer (MS) equipped with a Z-spray interface as described previously (Lepine and Deziel, 20111, Methods Mol Biol, 692: 61-69). Nitrogen was used for drying and argon was used as collision gas in multiple reactions monitoring (MRM) mode. HPLC (1100 HP) was equipped with a 4.6×150mm Eclipse XDB C8 column (Agilent) and the MS was connected to the HPLC through a T splitter (Valco). The third output of the splitter was fitted with a tube of internal diameter and length such that only 10% of the initial flow goes to the electrospray probe. Solvent A: ultrapure water containing 1% ACS grade acetic acid. Solvent B: acetonitrile (HPLC grade), containing 1% ACS grade acetic acid. The solvent gradient for the chromatographic runs was as follows: from 0 to 1 min 70% solvent A; from 1 to 13 min 100% solvent B; from 13 to 23 min 100% solvent B; from 23 to 25 min 70% solvent A; from 25 to 28 min 70% solvent A. Flow rate was set at 400 µL min-1 split to 40 µL min-1 by the T splitter. The MS parameters were: positive mode; needle voltage 3.0 kV; cone 30 V; block temperature 120°C and drying gas 150°C; nebulising gas 20 µL min-1 and drying gas 200 µl min-1. In full scan mode, the scanning range was set to m/z 100-400.

### EXAMPLE X

### β-galactosidase assay for LacZ expression

*β*-galactosidase activity was measured as described by Miller (1972, Immunochemistry, 9: 217-228), with slight modifications. Briefly, cells were grown in TSB without and with cerPAC to various cell densities. Samples of cell culture were retrieved at different time points and diluted in Z-buffer (Na₂HPO₄ 0.06 M; NaH₂PO₄ 0.04 M; KCl 0.01 M; MgSO₄.7H₂O 0.001 M; β-mercaptoethanol 0.05 M; pH 7.0). Cells in Z-buffer were permeabilized by the addition of one drop of 0.1% SDS and two drops of chloroform. Then, 200 µL of 4 mg mL⁻¹ ONPG was added to each reaction mixture, and enzyme reaction was stopped using 200 µL of 1 M Na₂CO₃. Cell debris were separated by centrifugation at 14,000 × g for 30 sec and color development was monitored at 420 nm. β-galactosidase activity was expressed in Miller units (MU), calculated as follows: 1,000×OD₄₂₀/T (min)×V (mL)× OD₆₀₀.

### EXAMPLE XI

### Antagonists/agonists assay

To evaluate the activity of cerPAC as antagonists/agonists against the natural AHL ligand of LasR or RhIR, 3-oxo-C₁₂-HSL (Sigma-Aldrich) and C₄-HSL (Cayman Chemical) were used as inducers in this assay. The AHL-deficient strain that has been engineered to produce β-galactosidase upon activation of LasR by 3-oxo-C₁₂-HSL [Δ*lasI* (*lasI-lacZ;* pME3853)] and RhlR by C₄-HSL [*ΔrhlI (rhI-lacZ;* pME3846)], were grown overnight in TSB medium. The overnight culture was diluted in fresh TSB and was grown to achieve an OD₆₀₀ = 0.3. An appropriate amount of sterilized cerPAC stock solution prepared in MilliQ water was added to sterile culture tube containing TSB. For control condition, either 3-oxo-C₁₂-HSL or C₄-HSL (stock solution in DMSO as a control) was added to sterile culture tube containing TSB, final DMSO concentration (after addition of cells) did not exceed 0.5 % v/v. Bacterial cells were added to TSB (final OD₆₀₀ = 0.05) without and with cerPAC approximately 30 min prior to the addition of the AHL at a final concentration of 3.13-50 nM (for 3-oxo-C₁₂-HSL) or 62.5-1000 nM (for C₄-HSL), to achieve final volume of 2 mL. Culture tubes were incubated at 37 °C for 3 h under shaking at 200 rpm, measurement of cell OD₆₀₀ and β-galactosidase assay were performed at the regular time intervals after 3 h of incubation. The concentration of cerPAC that reduced or increased the β-galactosidase activity compared to controls containing 3-oxo-C₁₂-HSL or C₄-HSL (without cerPAC) was considered to determine antagonist or agonist activity.

### EXAMPLE XII

### In silico docking analysis

To understand the interaction between components of the cerPAC with LasR and Lasl protein structures, a virtual docking was performed using the Piecewise Linear Potential and Lennard-Jones algorithms that can identify steric and hydrogen bonding interactions, and the Coulomb potential for electrostatic forces. *In silico* docking analysis was performed using the Molegro Virtual Docker 6.0 suite without the incorporation of water molecules. To maintain the search robustness, twenty rounds of iteration were used for each docking process. The S-adenosyl L methionine (NCBI Pubchem CID 34756) and the 3-oxo-C₁₂-HSL (NCBI Pubchem CID 127864) molecular structures were used as native ligand molecules, for the Lasl (RCSB Protein data base ID 1RO5) and LasR (RCSB Protein data base ID 2UV0) proteins, respectively. The components of cerPAC, epicatechin (NCBI PubChem CID 182232) and proanthocyanidin (NCBI PubChem CID 108065) molecular structures were used as ligands in virtual docking for both proteins. The MolDock search tool, that combines guided differential evolution and a cavity prediction algorithm was used for docking scores (in kcal mol⁻¹) based on the interaction energies of each complex. The best five positions with high Moldock interaction energies were sampled and compared in every complex computed. The Computed Atlas of Surface Topography of proteins was used to explore the volumes of the cavities in the target proteins. The molecular graphics and analyses were performed using the UCSF Chimera version 1.1.

### EXAMPLE XIII

### Growth kinetics

To assess growth kinetics, *P. aeruginosa* PA14 was grown in the absence or presence of cerPAC at 6.25, 25, 100, 200 and 300 µg mL⁻¹. An overnight culture of PA14, grown at 37°C with shaking at 200 rpm, was diluted 1,000-fold with TSB medium. This cell suspension containing approximately 10⁶ cells mL⁻¹ was aliquoted into sterile 100-well honeycomb microplates containing different amount of cerPAC and incubated at 37°C until stationary phase was reached. The OD₆₀₀ was recorded at 30 minutes time intervals using a BioScreen C system (Growth Curves USA, Piscataway, NJ). Each condition was set-up in four replicates. The optimum concentration of cerPAC that did not hinder growth of PA14 in TSB was selected in all subsequent assays, unless otherwise noted. Similarly, another set of experiments was conducted with larger volume (3 mL) to analyze the effect of cerPAC on growth and AHL production. Samples were collected at different time points for OD₆₀₀ and LC/MS analyses. Dry weight of the bacterial suspensions at each time point was determined using pre-weighed aluminum cups that were incubated at 65 °C for 4 h to allow water evaporation. Cups were weighed again to determine total dry weight.

### EXAMPLE XIV

### Bacterial cell enumeration on filters during the fly feeding assay

Enumeration of viable bacteria on filter during the infection period was completed using separate test vials inoculated with PA14 and uninoculated control sucrose vials that were sampled on alternative days, up to 12 days during infection period. Briefly, filters from the test vials were removed in sterile environment, placed in 50 mL polypropylene tube containing 5 ml of LB broth and vortexed for 30 sec. This LB medium containing the sampled bacterial cells was serially diluted in phosphate buffer saline solution (pH 7), and 30 µL was plated onto TSB agar and *Pseudomonas* Isolation agar (Thermo Scientific Remel, Fisher Scientific, Canada). Colonies were enumerated after incubation at 37 °C for 24 h.

### EXAMPLE XV

### Tetracycline membrane transport assay

Tetracycline membrane transport was assayed with some modifications (Dockter et al., 1978, Proc Natl Acad Sci USA, 75: 1319-1323; Dockter et al., 1975, Arch Biochem Biophys, 168: 81-88) by monitoring the fluorescence enhancement of tetracycline when it enters the cell. Bacterial culture in MHB-II were grown to OD₆₀₀=2, inoculated in fresh media and grown to OD₆₀₀=0.6. Cells were pelleted at 5000 rpm for 5 minutes and resuspended in 1:4 volume of 10 mM HEPES buffer pH 7.2. Tetracycline and cPACs samples were pipetted into the black non-transparent microtitre well plate (Falcon, Fisher Scientific, Canada) to adjust assigned concentration in 10 mM HEPES buffer pH 7.2. Cell suspension was pipetted and the fluorescence read at initial point and after 60 minutes at room temperature. Fluorescence at excitation and emission wavelengths of 405 and 535 nm, respectively were monitored.

### EXAMPLE XVI

### Gene expression analysis

Bacterial cells were grown to an OD600 of 0.5 to 0.8 (37°C, 150 rpm shaking) in MHB-II broth with or without different concentrations of cPAC RNA isolation, cDNA preparation and qRT-PCR were performed as mentioned earlier (Maisuria et al., 2015, Appl Environ Microbiol, 81: 3782-3792). Data were normalized to the endogenous reference gene of respective strains. The threshold cycle method (2^{-ΔΔCT}) (Livak and Schmittgen, 2001, Methods, 25: 402-408) used to analyze changes in gene expression in a given sample relative to the control (cells grown under the same conditions without cPAC).

## Claims

1. A synergistically active composition comprising an enriched polyphenolic cranberry extract comprising approximatively 95% proanthocyanidins (cPAC), and
- fosfomycin, tetracycline, trimethoprim, nitrofurantoin or azithromycin for use in treating a *P*. *mirabilis* urinary tract infection;
- azithromycin, sulfamethoxazole, nitrofurantoin or fosfomycin for use in treating *P. aeruginosa* PA01 urinary tract infection;
- kanamycin, tetracycline, azithromycin, nitrofurantoin or sulfamethoxazole for use in treating *P. aeruginosa* PA14 urinary tract infection; or
- azithromycin, tetracycline, kanamycin or nitrofurantoin for use in treating E. *coli* urinary tract infection.

2. The composition for uses according to claim 1, wherein the cranberry extract comprises proanthocyanidins, flavanols, anthocyanidins, procyanidins, terpenes, hydroxybenzoic acids, hydroxycinnamic acids, flavonoids, tannins, phenolic acids, other bioactive molecules or combinations thereof.

3. The composition for uses according to claim 1 or 2, wherein the cranberry extract is from at least one of *Vaccinium macrocarpon, Vaccinium oxycoccos, Vaccinium microcarpum,* and *Vaccinium erythrocarpum.*

4. The composition for uses according to any one of claims 1-3, wherein the cranberry extract is from *Vaccinium macrocarpon.*

5. The composition for uses according to any one of claims 1-4, wherein
- *E. coli is E. coli* CFT073;
- *P. mirabilis* is *P. mirabilis* HI4320.

## Patentansprüche

1. Synergistisch wirksame Zusammensetzung, die einen angereicherten polyphenolischen Cranberry-Extrakt umfasst, der etwa 95 % Proanthocyanidine (cPAC) umfasst, und
- Fosfomycin, Tetracyclin, Trimethoprim, Nitrofurantoin oder Azithromycin zur Verwendung bei der Behandlung einer *P. mirabilis*-Harnwegsinfektion;
- Azithromycin, Sulfamethoxazol, Nitrofurantoin oder Fosfomycin zur Verwendung bei der Behandlung einer *P. aeruginosa* PA01-Harnwegsinfektion;
- Kanamycin, Tetracyclin, Azithromycin, Nitrofurantoin oder Sulfamethoxazol zur Verwendung bei der Behandlung einer *P. aeruginosa* PA14-Harnwegsinfektion; oder
- Azithromycin, Tetracyclin, Kanamycin oder Nitrofurantoin zur Verwendung bei der Behandlung einer *E. coli*-Harnwegsinfektion.

2. Zusammensetzung zur Verwendung nach Anspruch 1, wobei der Cranberry-Extrakt Proanthocyanidine, Flavanole, Anthocyanidine, Procyanidine, Terpene, Hydroxybenzoesäuren, Hydroxyzimtsäuren, Flavonoide, Tannine, Phenolsäuren, andere bioaktive Moleküle oder Kombinationen davon umfasst.

3. Zusammensetzung zur Verwendung nach Anspruch 1 oder 2, wobei der Cranberry-Extrakt von mindestens einem aus *Vaccinium macrocarpon, Vaccinium oxycoccos, Vaccinium microcarpum* und *Vaccinium erythrocarpum* stammt.

4. Zusammensetzung zur Verwendung nach einem der Ansprüche 1-3, wobei der Cranberry-Extrakt aus *Vaccinium macrocarpon* stammt.

5. Zusammensetzung zur Verwendung nach einem der Ansprüche 1-4, wobei
- *E. coli E. coli* CFT073 ist;
- *P. mirabilis P. mirabilis* HI4320 ist.

## Revendications

1. Composition synergiquement active comprenant un extrait de canneberge polyphénolique enrichi comprenant environ 95% de proanthocyanidines (cPAC), et
- de la fosfomycine, de la tétracycline, du triméthoprime, de la nitrofurantoïne ou de l'azithromycine pour une utilisation dans le traitement d'une infection des voies urinaires par *P. mirabilis ;*
- de l'azithromycine, du sulfaméthoxazole, de la nitrofurantoïne ou de la fosfomycine pour une utilisation dans le traitement d'une infection des voies urinaires par *P. aeruginosa* PA01 ;
- de la kanamycine, de la tétracycline, de l'azithromycine, de la nitrofurantoïne ou du sulfaméthoxazole pour une utilisation dans le traitement d'une infection des voies urinaires par *P. aeruginosa* PA14 ; ou
- de l'azithromycine, de la tétracycline, de la kanamycine ou de la nitrofurantoïne pour une utilisation dans le traitement d'une infection des voies urinaires par E. *coli.*

2. Composition pour utilisations selon la revendication 1, dans laquelle l'extrait de canneberge comprend des proanthocyanidines, des flavanols, des anthocyanidines, des procyanidines, des terpènes, des acides hydroxybenzoïques, des acides hydroxycinnamiques, des flavonoïdes, des tannins, des acides phénoliques, d'autres molécules bioactives ou des combinaisons de ceux-ci.

3. Composition pour utilisations selon la revendication 1 ou 2, dans laquelle l'extrait de canneberge provient d'au moins l'un parmi *Vaccinium macrocarpon, Vaccinium oxycoccos, Vaccinium microcarpum* et *Vaccinium erythrocarpum.*

4. Composition pour utilisations selon l'une quelconque des revendications 1 à 3, dans laquelle l'extrait de canneberge provient de *Vaccinium macrocarpon.*

5. Composition pour utilisations selon l'une quelconque des revendications 1 à 4, dans laquelle
- *E. coli* est *E*. *coli* CFT073 ;
- *P. mirabilis* est *P. mirabilis* HI4320.
